# EUROPEAN PATENT SPECIFICATION

(11) **EP 2 583 696 B1**
(45) Date of publication and mention of the grant of the patent: **21.02.2018**
(21) Application number: 12189143.6
(22) Date of filing: 18.10.2012
(51) Int. Cl.: A61L 9/01, C07C 69/675, C07C 205/44, C07C 223/06, C07C 323/22, C07C 229/08, C08B 30/00

(54) **Low volatile reactive malodor counteractives and methods of use thereof**
Schwerflüchtige reaktive Geruchsgegenmittel und Verwendungsverfahren dafür
Agents contre les mauvaises odeurs de réactifs faiblement volatils et leurs procédés d'utilisation

(30) Priority: 20.10.2011 US 201113277288
(43) Date of publication of application: 24.04.2013
(73) Proprietor: International Flavors & Fragrances Inc., New York, NY 10019 (US)
(72) Inventor: PLUYTER, Johan Gerwin Lodewijk, Lindenhurst, Illinois 60046 (US); SASAKI, Takashi, Morganville, New Jersey 07751 (US); HUANG, Xiao, Freehold, NJ 07728 (US)
(74) Representative: Lawrence, John

(56) References cited:
- EP-A1- 2 272 491
- EP-A1- 2 412 677
- WO-A1-02/051788
- WO-A1-2005/021051
- WO-A1-2006/076821
- WO-A1-2007/012586
- WO-A1-2011/020116
- WO-A2-01/36362
- WO-A2-99/00114
- GB-A- 735 693
- GB-A- 1 373 609
- US-A- 2 475 273
- US-A- 2 962 478
- US-A- 3 580 906
- US-A- 5 601 809
- US-A1- 2003 124 157
- US-A1- 2004 209 269
- US-A1- 2007 225 485
- US-A1- 2008 242 721
- US-A1- 2010 111 889
- US-A1- 2011 070 181
- US-B1- 6 630 128
- KAHOVEC, JAROSLAV: "Aldehyde Functionalization of Styrene Polymers", POLYMER BULLETIN, vol. 4, no. 12, 1 June 1981 (1981-06-01), pages 731-733, XP002690309, Berlin(DE) ISSN: 1436-2449

## Description

### Background of the Invention

Malodors are offensive odors, which are encountered in the air and on many substrates such as fabrics, hard surfaces, skin, and hair. Amines, thiols, sulfides, short chain aliphatic and olefinic acids, *e.g.,* fatty acids, are typical of the chemicals found in and contributed to sweat, household, and environmental malodors. These types of malodors typically include indole, skatole, and methanethiol found in toilet and animal odors; piperidine and morpholine found in urine; pyridine and triethyl amine found in kitchen and garbage odors; and short chain fatty acids, such as 3-methyl-3-hydroxyhexanoic acid, 3-methylhexanoic acid or 3-methyl-2-hexenoic acid, found in axilla malodors. Compounds which have been found in the axilla are described for example by Zeng, et al. ((1991) J. Chem. Ecol. 17:1469-1492).

Malodor counteractants or masking agents have been described in the art. For example, sulfhydryl reactants, such as diethyl fumarate, di-n-butyl maleate and N-ethylmaleimide are disclosed in US 5,601,809 as compounds that are effective against axillary malodor. Further, the use of certain aromatic unsaturated carboxylic acid esters in combination with alkyl fumarates as malodor counteractants is disclosed in US 6,610,648. US 6,403,075 addresses fragrance materials with a phenyl ring moiety as ammonia masking agents. Similarly, US 2002/0058017 describes cis-3-hexenol to mask ammonia. Moreover, US 7,585,833 describes methods for formulating fragrances to mask malodor present in products containing ammonia and substituted amines (See, US 6,379,658, US 6,376,741, US 5,769,832, and US 5,037,412).

Although the art describes compositions and methods for neutralizing certain malodors, there still remains a need for additional compounds that are more efficient against malodors. In addition, these new materials possess very low volatility such that the overall olfactory character of the fragrance is not impacted or only to a small extent.

### Summary of the Invention

The present invention features a malodor counteractant compound comprising a benzaldehyde covalently attached to a polymer or a solid surface, wherein the compound is as defined in claim 1. Thus when the benzaldehyde is attached to a polymer, the compound is

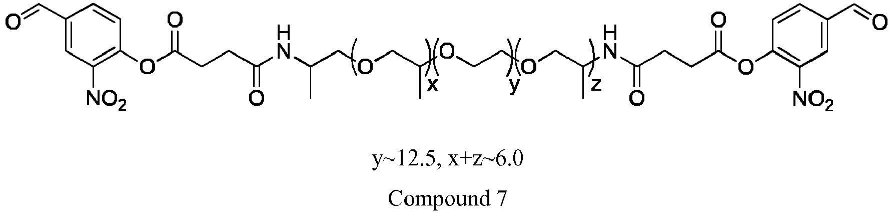

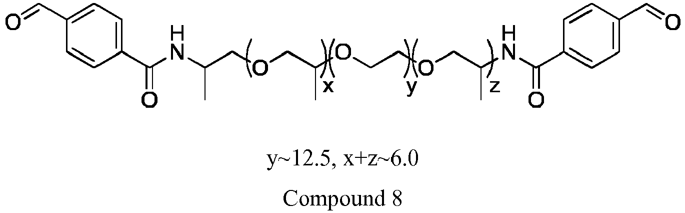

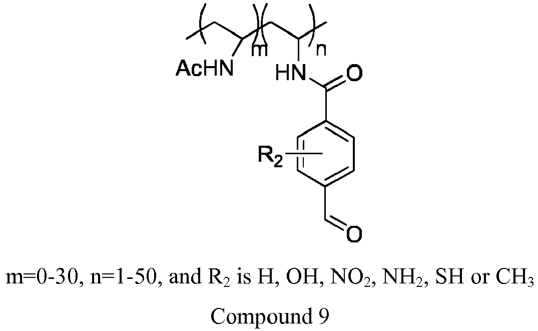

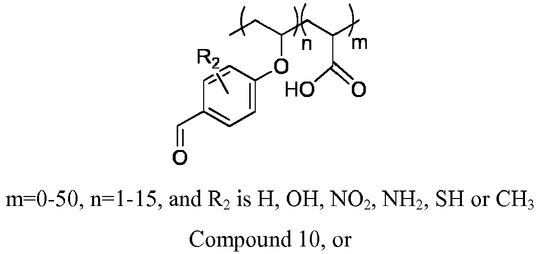

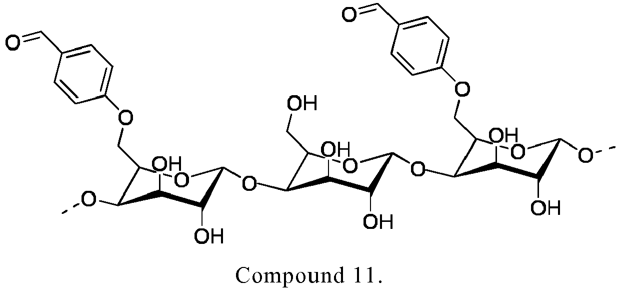

The invention also provides consumer, industrial or textile products as defined in claim 3. Thus consumer and industrial products containing the above-defined malodor counteractant are provided, as are textile products comprising a malodor counteractant compound comprising a benzaldehyde covalently attached to a polymer, a surfactant or a solid surface, wherein the polymer comprises a polyol, polysaccharide, polyamine, polyacrylate, alkene oxide polymer with OH or NH₂ end groups, or block or random copolymer thereof; wherein the surfactant comprises a poloxamine or an unbranched C13-C15 oxo alcohol; or wherein the solid surface comprises a silica or clay surface.

The invention further provides methods for using a malodor counteractant to counteract amine-based malodor, wherein the method is as defined in claim 5. Thus the method comprises the step of adding a malodor counteractant compound to a consumer, industrial or textile product so that the amine-based malodor of the product or the surrounding environment thereof is counteracted, wherein the malodor counteractant compound comprises a benzaldehyde covalently attached to a polymer, a surfactant or a solid surface, wherein the polymer comprises a polyol, polysaccharide, polyamine, polyacrylate, alkene oxide polymer with OH or NH₂ end groups, or block or random copolymer thereof.

The malodor counteractant compounds for use according to the invention are defined in claims 1, 3 and 5 respectively. According to certain aspects of the present invention, the malodor counteractant compound has the structure: 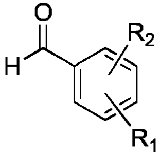 wherein R₁ is a polymer, a surfactant, or a solid surface; and R₂ is H, OH, NO₂, NH₂, SH or CH₃.

In some embodiments, the polymer is a polyol, polysaccharide, polyamine, polyacrylate, alkene oxide polymer with OH or NH₂ end groups, or block or random copolymer thereof; the surfactant is a poloxamine or an unbranched C₁₃-C₁₅ oxo alcohol; and the solid surface is a silica or clay surface.

In specific embodiments of this invention, the polymer is (a) polyalkylene oxide with OH or NH₂ end groups such as polyethyleneoxide, polypropyleneoxide, polytetrahydrofuran, polyetheramine, a block or random copolymer variant thereof such as PLURONICS or SYNPERONICS, or a branched copolymer such as a TETRONIC polymer; (b) polyvinyl amine or a copolymer with vinyl formamide or vinyl acetamide; (c) poly vinyl alcohol or a copolymer with vinyl acetate, olefin such as ethylene and propylene, or acrylate; or (d) polysaccharide (*i.e.,* maltodextrin, starch, guar, xanthan, carboxymethyl cellulose, hydroxyethyl cellulose, carrageenan, or cationic/amphoteric/hydrophobically substituted polysaccharide).

### Detailed Description of the Invention

The present invention provides compounds composed of a benzaldehyde covalently attached to certain polymers or to a solid surface (e.g. silica or clay), for use as malodor counteractants, and provides consumer and industrial products that contain said compound. Textile products that contain a malodor counteractant compound composed of a benzaldehyde covalently attached to certain polymers, a surfactant or a solid surface (which is silica or clay) are likewise provided.

Advantageously, the benzaldehyde of the malodor counteractant compounds contains at least one aldehyde group, which is capable of binding or reacting with amine-based malodors, thereby effectively reducing the concentration of these malodors in consumer, industrial or textile products. Moreover, the compounds of the present invention have a low vapor pressure such that the compounds can be added in significant quantities to products without impacting the olfactory character of the products. Given these features, the compounds of the present invention find use as additives to consumer products to reduce the concentration of malodors in the headspace of the product. Furthermore, the compounds of the present invention can be used to form a fragrance or flavor encapsulate or other delivery system such that while the delivery system is delivering its payload, malodors are removed from the air. Alternatively, the compounds of the present invention can be formulated into a product, such as a fragrance, which can be optionally formulated into a delivery system.

As indicated, particular embodiments feature compounds with low-to-no vapor pressure. Vapor pressure (P^{O}) is the pressure of a vapor of a compound in equilibrium with its pure condensed phase (solid or liquid). Vapor pressure is measured in the standard units of pressure. The International System of Units (SI) recognizes pressure as a derived unit with the dimension of force per area and designates the pascal (Pa) as its standard unit. One pascal is one Newton per square meter (N·^{m-2} or kg·^{m-1•s-2}). Vapor pressures depend on the temperature and vary with different compounds due to differences in molecule-molecule interactions. For example, vapor pressure at 25°C of n-alkanes is a function of chain length, wherein larger n-alkane molecules have lower P^{O} due to greater polarizability and increased strength of London Dispersion intermolecular forces. The vapor pressure of a compound can be determined by conventional methods known to those of skill in the art. In particular embodiments, compounds of the present invention have a vapor pressure of less than 200 Pa (1.5 mmHg), less than 100 Pa (0.75 mmHg), less than 50 Pa (0.375 mmHg), less than 20 Pa (0.15 mmHg), or less than 10 Pa (0.075 mmHg), at 25°C.

The benzaldehyde of the malodor counteractant compounds of the present invention is, in particular embodiments a functionalized benzaldehyde. In more particular embodiments, the benzaldehyde counteractant compound, has the structure: 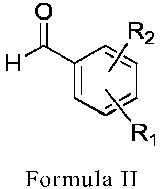 wherein
R₁ is a polymer, a surfactant, or a solid surface, as appropriate, and according to the definitions as given in the claims; and
R₂ is H, OH, NO_{2,} NH₂, SH or CH₃.

Formula II is intended to include any *p-, m*-*,* or *o*- functionalized benzaldehyde, wherein R₁ is a functionalization point for modulating log p and hydrogen bonding, as well as a linker to polymers, etc. In addition to a single attachment point, the -R₁ group can have multiple sites. These multiple attachment points can also be connected to each other in small ring systems or anchoring points on a polymer or a substrate.
A polymer in accordance with the present invention is a molecule composed of repeating monomer units. In contrast to a polymer, which can contain numerous monomers, an oligomer is a molecule that is composed of a few monomer units. In this respect, oligomers include dimers, trimers, tetramers, and the like.

The polymers for use according to the invention are defined in claims 1, 3 and 5 respectively. According to certain aspects of the present invention, a polymer can be a polyol (*e.g.,* polyvinyl alcohol or a copolymer with vinyl acetate, olefin such as ethylene and propylene, or acrylate); polysaccharide (*e.g.,* maltodextrin, starch, guar, xanthan, carboxymethyl cellulose, hydroxyethyl cellulose, carrageenan, or cationic/amphoteric/hydrophobically substituted polysaccharide); polyamine (*e.g.,* polyvinyl amine or a copolymer with vinyl formamide or vinyl acetamide); polyacrylate, optionally with alcohol groups; and polyalkylene oxides with OH or NH₂ end groups, including, *e.g.,* polyetheramine (JEFFAMINES) or block or random copolymers thereof, e.g. block or random copolymer variants of polyethyleneoxide, polypropyleneoxide, polytetrahydrofuran (*e.g.,* PLURONICS/SYNPERONICS). The polyalkylene oxide of the present invention may be an alkyl ether having from 4 to 25, preferably 4 to 16, moles of ethylene oxide per mole of alkyl phenol (*e.g.,* polyethylene glycol (PEG), polyethyleneoxide (PEO), polypropyleneoxide, and polytetrahydrofuran) and a block or random copolymer variant thereof or a branched copolymer such as a TETRONIC polymer.

The surfactants for use according to the invention are defined in claims 3 and 5 respectively. A surfactant of the present invention is a compound that lowers the surface tension of a liquid or the interfacial tension between two liquids or between a liquid and a solid. A surfactant may act as a detergent, wetting agent, emulsifier, foaming agent, or dispersant. A surfactant is usually an organic compound that is amphiphilic. Surfactants include molecules such as PLURONIC surfactants (based on ethylene oxide, propylene oxide and/or butylenes oxide as di- and tri-block copolymers) and TETRONIC surfactants (poloxamine or block copolymers based on ethylene oxide and propylene oxide with a vapor pressure of <0.1 mmHg at 25°C) including TETRONIC 901, TETRONIC 701, TETRONIC 90R4, and TETRONIC 904, and LUTENSOL AO nonionic surfactants (unbranched C₁₃-C₁₅ oxo alcohol) including LUTENSOL AO3, LUTENSOL AO4, LUTENSOL AO5, and LUTENSOL AO7.

The solid surfaces for use according to the invention are defined in claims 1, 3 and 5 respectively. In certain aspects of the present invention, a solid surface includes, but is not limited to, a silica surface (*e.g.,* a synthetic amorphous silica surface such as SYLOID), clay or other solid mineral materials with an appropriate functional group to attach the benzaldehyde moiety. In another embodiment, the solid surface is the surface of a delivery system such as a nanoparticle, microparticle, nanocapsule or microcapsule, which attaches to one or more benzaldehyde moieties.

The malodor counteractant compound of this invention has at least one aldehyde reactive group for reacting or binding amine-based malodors. In some embodiments, the malodor counteractant compound of this invention has multiple hydrogen-bonding (acceptor) sites for reacting or binding amine-based malodors.

The malodor counteractant compounds for use according to the invention are defined in claims 1, 3 and 5 respectively. According to certain aspects of the present invention, specific examples of malodor counteractant compounds containing a benzaldehyde of Formula II include, but are not limited to, the following examples:

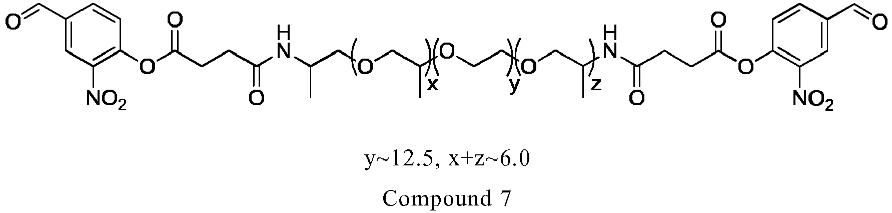

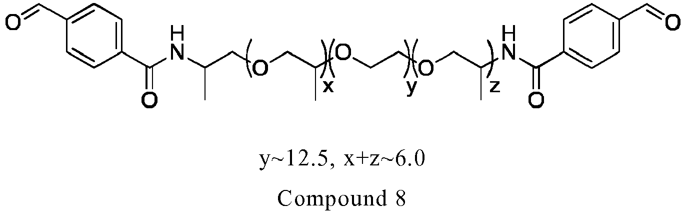

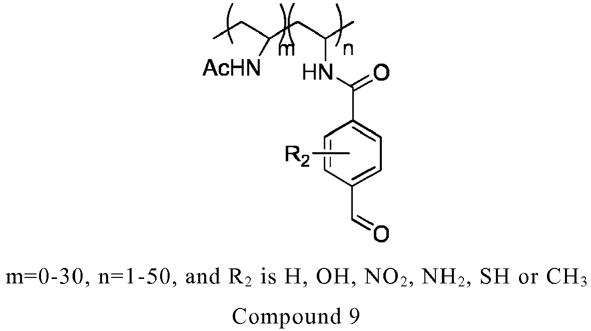

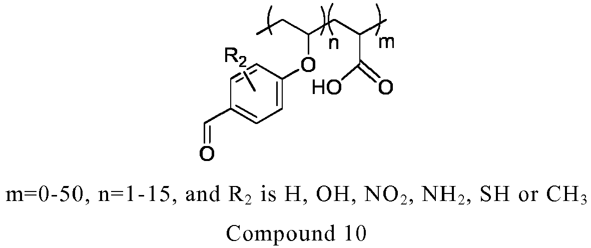

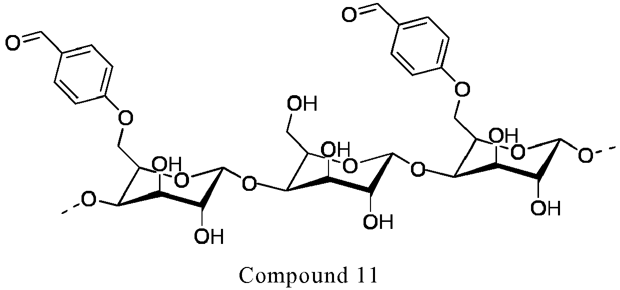

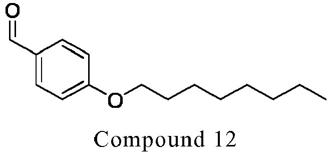

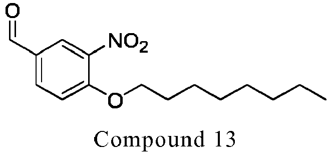

Malodor counteractant compounds of the present invention are produced by covalently attaching a benzaldehyde as described herein to a polymer, a surfactant, or a solid surface, as appropriate. Given that the benzaldehyde is covalently attached, this moiety is not released before or during use in a consumer, industrial or textile product, *e.g.,* the compounds of the present invention are not pro-fragrances. Specific examples of reagents and reactions conditions for preparing the compounds of the present invention are provided in Examples.

The malodor counteractant compounds of the present invention can be used in a variety of forms and in a variety of products. Advantageously, the compounds of the present invention are reactive against potent malodor ingredients while not affecting the odor of a fragrance or final product. Furthermore, these compounds and the methods herein can be pursued in any situation where malodor is present. In this respect, the present invention also features a method for counteracting amine-based malodor of consumer, industrial and textile products, as well as the surrounding environment, by introducing or adding one or more malodor counteractant compounds of the present invention to a consumer, industrial or textile product so that the amine-based malodor of the product is counteracted, as defined in claim 5.

Benzaldehyde malodor counteractant compounds of the present invention may react with amine-based malodor molecules, for example, but not limited to, according to the following schemes: 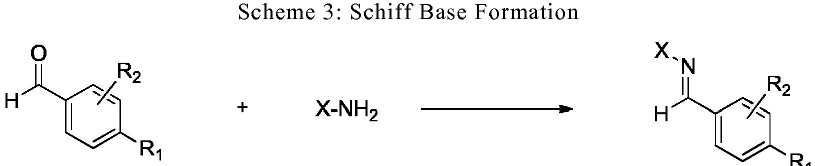 wherein R₁ is a polymer, a surfactant, or a solid surface; R₂ is H, OH, NO₂, NH₂, SH or CH₃; and X-NH₂ represents an amine-based malodor molecule; 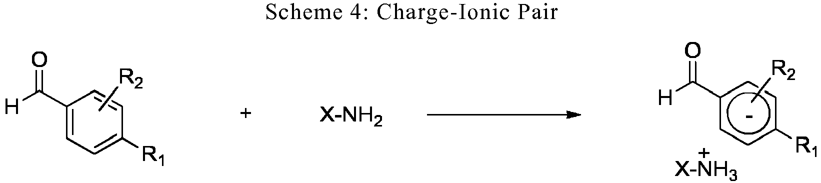 wherein R₁ is a polymer, a surfactant, or a solid surface; R₂ is H, OH, NO₂, NH₂, SH or CH₃; and X-NH₂ represents an amine-based malodor molecule; and 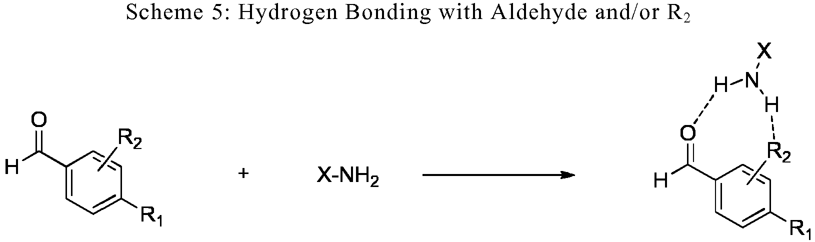 wherein R₁ is a polymer, a surfactant, or a solid surface; R₂ is H, OH, NO₂, NH₂, SH or CH₃; and X-NH₂ represents an amine-based malodor molecule.

For the purposes of the present invention, a compound counteracts a malodor if it measurably (either qualitatively or quantitatively) reduces the presence of a malodor. In particular embodiments, the malodor counteractant compounds of the present invention reduce the presence of amine-based malodor of a product by 50-100% as compared to a product that does not have the malodor counteractant compounds.

Malodors particularly targeted by the compounds of the present invention include amine-based malodor such as bathroom odors, sweat, food odors, textile odors, home care and personal care product base odors, adhesive odors, and paint odors. In this respect, the compounds of the present invention can be used in air refresheners, fabric refresheners, bar soaps, perfumes, fragrances, cologne, bath or shower gels, shampoos or other hair care products, cosmetic preparations, body odorants, deodorants, antiperspirants, liquid or solid fabric detergents or softeners, bleach products, disinfectants or all-purpose household or industrial cleaners, food, or industrial or textile products such as adhesives, paints, coatings, or textiles. In yet another embodiment, one or more of the compounds of the present invention are used as part of a delivery system or polymer system to deliver a fragrance or compound of interest (*e.g.,* a pharmaceutical).

The following are provided as specific embodiments of the present invention. Other modifications of this invention will be readily apparent to those skilled in the art. The scope of the invention is defined by the claims. All reagents were purchased from Sigma-Aldrich, Inc. unless otherwise noted. Further, as used herein all percentages are weight percent unless otherwise noted, ppm is understood to be parts per million, L is understood to be liter, mL is understood to be milliliter, µL is understood to be microliter, mol is understood be mole, mmol is understood be millimole, and M is understood to be moles per L. IFF as used in the examples is understood to mean International Flavors & Fragrances Inc., New York, NY, USA.

The invention will be further described in the following examples, which do not limit the scope of the invention described in the claims.

### Reference Example 1: Preparation of Levulinic Acid Chloride

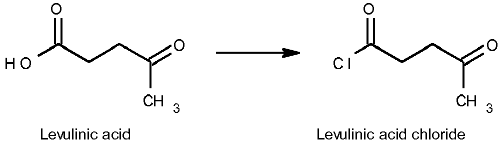

Oxalyl chloride in dichloromethane (2 M, 450 mL, 0.9 mol) was added drop-wise to a stirring mixture of levulinic acid (100 g, 0.857 mol) and dimethylformamide (DMF, 2 mL) for about 1.5 to 2 hours at room temperature. After the addition was completed, the resulting mixture was stirred for an additional 0.5 hours. Solvents were removed *in vacuo* and the crude mixture was used in subsequent reactions.

### Reference Example 2: Preparation of Levulinic TETRONICS (Compound 2)

Triethylamine (6.91 mL, 49.50 mmol) was added to a stirring mixture of TETRONIC 901 (23.5 g, 24.77 mmol) dissolved in dichloromethane (150 mL) under nitrogen atmosphere at 0-5°C. Levulinic acid chloride (prepared as in Example 1) was then added drop-wise. The resulting mixture was warmed to room temperature and stirred overnight. Triethylammonium chloride salt was subsequently filtered off. The organic layer was washed with saturated Na₂CO₃ and concentrated *in vacuo* to afford the product levulinate TETRONIC with greater than 90% functionalization as determined by NMR spectroscopy.

### Reference Example 3: Preparation of Levulinate MALTRIN QD M585 Maltodextrin

Triethylamine (12.5 g, 0.124 mmol) was added to a stirring mixture of MALTRIN QD M585 maltodextrin (16.2 g, 0.100 mol) dissolved in DMF (60 mL) under nitrogen atmosphere at 0-5°C. The mole ratios of maltodextrin herein were based on monomer molecular structure of the entire polymeric chain. Levulinic acid chloride (prepared as in Example 1) was then added drop-wise. The resulting mixture was warmed to room temperature and stirred overnight. The triethylammonium chloride salt was subsequently filtered off. Precipitate was collected with a mixture of tetrahydrofuran (THF)/isopropanol (IPA) at a volume ratio of 1:9 (THF:IPA). The solid was filtered, washed several times with the THF:IPA mixture, and dried under vacuum to afford the product levulinate MALTRIN QD M585 maltodextrin with about 15-20% functionalization as determined by NMR spectroscopy.

### Reference Example 4: Preparation of Levulinate LUTENSOL AO7

Triethylamine (11.7 g, 0.115 mol) was added to a stirring mixture of LUTENSOL AO7 (20.0 g, 57.5 mmol) dissolved in acetone (150 mL) under nitrogen atmosphere at 0-5°C. Levulinic acid chloride (prepared as in Example 1) was then added drop-wise.

The resulting mixture was warmed to room temperature and stirred for overnight. The triethylammonium chloride salt was filtered off. The solvent was concentrated *in vacuo* to afford the product levulinate LUTENSOL AO7 with greater than 85% functionalization as determined by NMR spectroscopy.

### Reference Example 5: Preparation of Octyl Levulinate (Compound 6)

I-Bromooctane (332 g, 1.72 mol) was added to a stirring mixture of levulinic acid (200 g, 1.72 mol), tetrabutylammonium bromide (55.1 g, 0.17 mol), and K₂CO₃ (357 g, 2.58 mol) in DMF (500 mL) drop-wise and stirred at room temperature for overnight. The resulting mixture was diluted in toluene and the organic layer was washed with HCl (10%), brine, and water. The solvent was concentrated *in vacuo* and the oil was purified by vacuum distillation to afford the product octyl levulinate as a clear oil.

### Example 6: Preparation of 4-Octyloxy Benzaldehyde

A mixture of 4-hydroxybenzaldehyde (10.0 g, 81.8 mmol), 1-bromooctane (15.8 g, 81.8 mmol), and K₂CO₃ (34.0 g, 0.25 mol) in DMF (150 mL) was heated to reflux for 8 hours and then cooled to room temperature. The reaction mixture was subsequently diluted in toluene. The organic layer was washed with HCl (10%), brine, and water. The solvent was concentrated *in vacuo* and the oil was purified by short silica gel plug (dichloromethane (DCM) in n-hexane, 10%). Solvent was dried *in vacuo* to afford the product 4-octyloxy benzaldehyde as a slightly colored oil.

### Example 7: General Synthesis of Benzaldehyde Malodor Counteractant Compounds

Benzaldehyde malodor counteractant compounds can be prepared from a variety of benzaldehydes which are mono-, di-, or tri-substituted with functional groups in the *p-*, *m*-, and/or *o*- positions. Exemplary benzaldehyde starting materials include, but are not limited to, 4-hydroxybenzaldehyde, 4-methoxybenzaldehyde, 4-bromobenzalehyde, 4-formylbenzonitrile, 3-methoxy-*p*-anisaldehyde, Vanillin, Isovanillin, Syringaldehyde, potassium 3-formylphenyltrifluroroborate, 3-chloro-4-hydroxybenzaldehyde, 3-formyl-4-hydroxybenzoic acid, 4-formylbenzoic acid, 3-fluoro-4-formylphenyboronic acid, 3-allysalicyladehyde, 4-ethyl-3-nitrobenzaldehyde, *p*-formylbenzoic acid *N*-hydroxysuccinimide ester, 4-[(tertbutyldimethylsilyl)oxy]benzaldehyde.

As will be readily appreciated by of one of skill in the art, a halogenated benzaldehyde can have any halogen group substituted at the same positions(s). For example, 3-chloro-4-hydroxybenzaldehyde can readily be used as can 3-fluoro-4-hydroxybenzaldehyde.

Benzaldehyde malodor counteractant compounds can be prepared using one or more of the following methods.

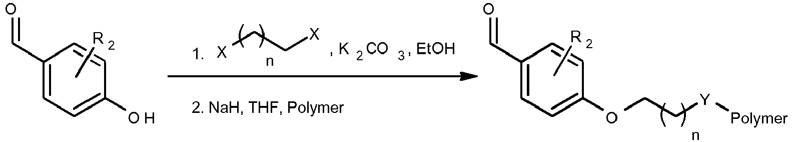

*Method 1.* Step 1 involves mono-alkylation of hydroxybenzaldehyde with di-halogenated alkane, where X is bromide, chloride, iodide. Step 2 involves conjugation of the polymer via the other halogen of the di-halogenated alkane and a hydroxy-terminated or primary amine-terminated polymer (Y-Polymer).

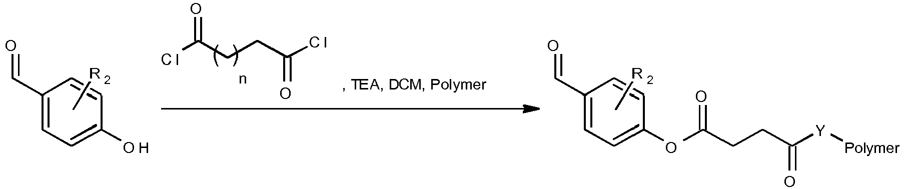

*Method 2.* This method involves incorporation of a hydroxy-terminated or primary amine-terminated polymer (Y-Polymer) via double esterification/amidation of hydroxyl- or amino-benzaldehyde with di-acid chloride crosslinker.

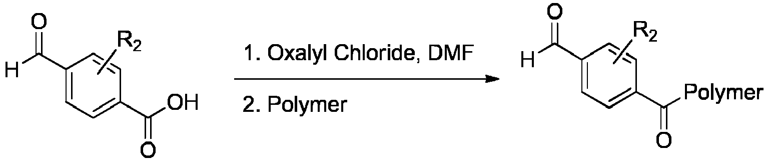

*Method 3.* Step 1 involves acid chloride formation with the carboxylic acid moiety of the benzaldehyde. Step 2 involves esterification or amidation with a hydroxy-terminated or primary amine-terminated polymer/material.

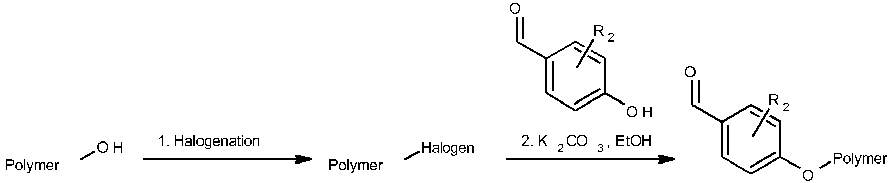

*Method 4.* Step 1 involves bromination or chlorination of a primary alcohol-terminated polymer. Step 2 includes alkylation of hydroxyl-benzaldehyde with the halogenated polymer.

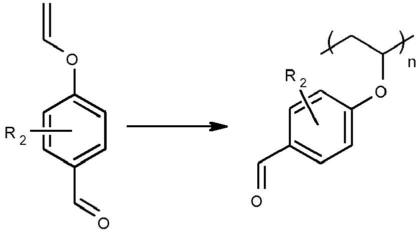

*Method 5.* This method involves polymerization or copolymerization of benzaldehyde functionalized monomer.

When hydrolysis stable reactive polymers, surfaces or surfactants are required, polymer/surface/surfactant can employ amide groups as their linkage. Besides using the acid chloride to create ester or amide bonds, N,N'-dicyclohexylcarbodiimide (DCC) coupling can be used to create these linkages. Moreover, esterification can be performed using any suitable acid with alkyl halides.

### Example 8: Synthesis of Dibenzaldehyde JEFFAMINE ED-900

To a stirring mixture of 4-formyl benzaldehyde (10.0 g, 0.130 mol) in THF (250 mL) with catalytic amount of DMF (12 mL) was added drop-wise, thionyl chloride (7.93g, 0.130 mol) at 0°C. After the complete addition, the mixture was stirred for an additional 0.5 hour. The synthesized acid chloride was then used in-situ for the following reaction. To a stirring mixture of JEFFAMINE ED-900 (27.3 g, 30.3 mmol) dissolved in THF (200 mL) was added triethylamine (6.74 g, 66.6 mmol) under nitrogen atmosphere at 0-5°C. Acid chloride was added drop-wise to the stirring mixture. After addition, the reaction mixture was warmed to room temperature and was stirred overnight. The triethylammonium chloride salt was filtered off. The organic layer was washed with saturated Na₂CO₃ and concentrated *in vacuo* to afford the dibenzaldehyde JEFFAMINE ED-900 with greater than 85% functionalization as determined by NMR spectroscopy.

### Example 9: Synthesis of 4-Octyloxy Benzaldehyde

A mixture of 4-hydroxybenzaldehyde (10.0 g, 81.8 mmol), 1-bromooctane (15.8 mmol, 81.8 mmol), and K₂CO₃ (34.0 g, 0.25 mol) in DMF (150 mL) was heated to reflux for 8 hours. After cooling the reaction back down to room temperature, the reaction mixture was diluted in toluene and the organic layer was washed with 10% HCl, brine, and water. After concentrating *in vacuo,* the oil was purified by short silica gel plug (10% DCM in n-hexanes). Solvent was dried *in vacuo* to afford the 4-octyloxy benzaldehyde as a slightly colored oil.

### Example 10: Synthesis of 4-Octyloxy-3-nitrobenzaldehyde

A mixture of 4-hydroxy-3-nitrobenzaldehyde (10.0 g, 81.8 mmol), 1-bromooctane (15.8 mmol, 81.8 mmol), and K₂CO₃ (34.0 g, 0.25 mol) in DMF (150 mL) was heated to reflux for 8 hours. After cooling the reaction back down to room temperature, the reaction mixture was diluted in toluene and the organic layer was washed with 10% HCl, brine, and water. After concentrating *in vacuo,* the oil was purified by short silica gel plug (10% DCM in n-hexanes). Solvent was dried *in vacuo* to afford the 4-octyloxy-3-nitrobenzaldehyde as a slightly colored oil.

### Example 11: Testing Procedure

The testing procedure described herein was applicable to malodor counteractant compounds containing water-soluble, modified polymers, oligomers, or surfactants.

A solution of n-butyl amine (*n*BA) in methanol (0.05% by weight, 500 ppm) was prepared and stored at 0°C in a refrigerator prior to use. A solution of a malodor counteractant compound to be tested was prepared (50 to 100 mL) with distilled (DI) water and thoroughly mixed with a magnetic stir bar. The *n*BA solution was added to the solution of the malodor counteractant compound and the molar ratio of the malodor counteractant compound and *n*BA was adjusted to 1:1. For a polymer-containing malodor counteractant compound, the averaged molecular weight of the polymer was used for this calculation. Water or diethyl phthalate (DEP) containing the same amount of *n*BA as the test group was used as a control.

For analysis, the aqueous polymer/surfactant (1 mL) was placed into a 20 mL headspace vial using a positive displacement pipette. *n*BA solution (0.05%, 250 µL) was subsequently added into the vial. The vial was then capped immediately. The vial was placed in a holder and set on an orbital incubator for a pre-selected mixing/equilibration time. An aliquot of headspace above the reaction solution was then sampled and injected into the gas chromatograph for separation and detection.

### Comparative Example 12: Evaluation of Malodor Counteractant Compounds with α-Keto Moiety

The ability of α-keto-containing molecules to reduce the amount of amine-based malodor was determined. Water was used as the control. The results are presented in Table 1.

**TABLE 1**

| α-Keto-Containing Molecules | Malodor Reduction |
|---|---|
| 2-Pentanon | 75% at 1 hour and 83% at 12 hours |
| Ethyl levulinate | 41 % at 1 hour and 69% at 12 hours |
| Butyl levulinate | 39% at 1 hour and 62% at 12 hours |
| Octyl levulinate (Compound 6) | 75% at 1 hour and 78% at 12 hours |

The malodor counteracting effect of malodor counteractant compounds containing an α-keto moiety covalently attached to a polymer including PEG-dilevulinate, TETRONIC 701 tetra-levulinate, and TETRONIC 901 tetraluvulinate were evaluated following the testing procedure described as above. The mixing/equilibration time was an hour. DEP was used as the Control. The results are presented in Table 2.

**TABLE 2**

| Malodor Counteractant Compounds | Malodor Reduction |
|---|---|
| PEG-dilevulinate | 4% |
| TETRONIC 701 tetra-levulinate | 97% |
| TETRONIC 901 tetraluvulinate | 75% |

### Example 13: Evaluation of Benzaldehyde Malodor Counteractant Compounds

The selective reactivity of *n*BA with certain small molecule benzaldehydes was determined to demonstrate benzaldehyde reactivity. The results of this analysis are presented in Table 3.

**TABLE 3**

| Ingredient | nBA reduction (T=I) | nPT reduction (T=I) | nBA reduction (T=+12) | nPT reduction (T=+12) |
|---|---|---|---|---|
| Acalea | 100.00 | 46.67 | 100.00 | 93.70 |
| Aubepine | 100.00 | 4.47 | | |
| Benzaldehyde | 100.00 | 12.22 | 100.00 | 11.09 |
| Isobutavan | 100.00 | 0.31 | 100.00 | 0.00 |
| Salicylaldehyde | 100.00 | 0.00 | 100.00 | 15.53 |

| | | | | |
|---|---|---|---|---|
| nBA = n-butylamine, nPT = 1-propanethiol. | | | | |

The reactivity of surfactant containing benzaldehyde malodor counteractant compounds, Compounds 12 and 13, was analyzed. The results of this analysis are presented in Table 4.

**TABLE 4**

| Benzaldehyde | nBA reduction (T=I) | nPT reduction (T=I) | nBA reduction (T=+12) | nPT reduction (T=+12) |
|---|---|---|---|---|
| Compound 12 | 100.00 | 7.63 | 100.00 | 10.85 |
| Compound 13 | 100.00 | 7.85 | 100.00 | 20.25 |

| | | | | |
|---|---|---|---|---|
| nBA = n-butylamine, nPT = 1-propanethiol. | | | | |

The absorptivity of malodor components by a polymer containing benzaldehyde malodor counteractant compound, dibenzaldehyde functionalized JEFFAMINE ED-900 (compound 8), was analyzed. The results of this analysis are presented in Table 5.

**TABLE 5**

| Malodor Ingredient | Percent Reduction |
|---|---|
| Hexanal | -48% |
| IVA | -91% |
| Octanal | -66% |
| Decanal | -79% |

## Claims

1. A malodor counteractant compound comprising a benzaldehyde covalently attached to:
a) a polymer, wherein the compound is 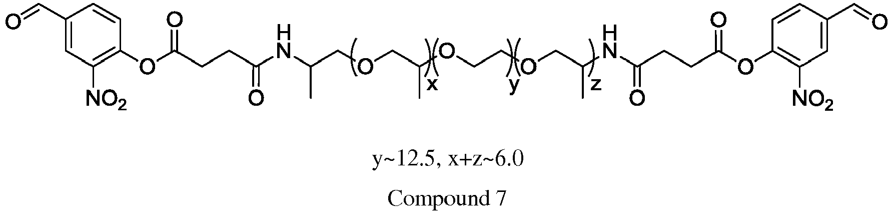 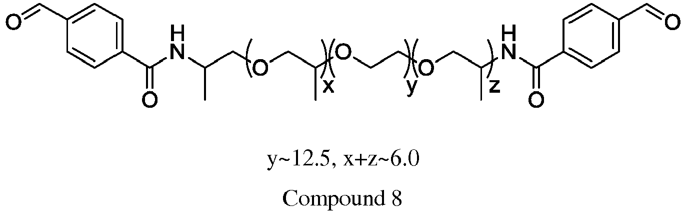 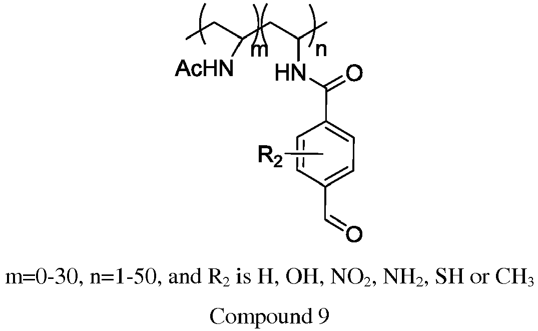 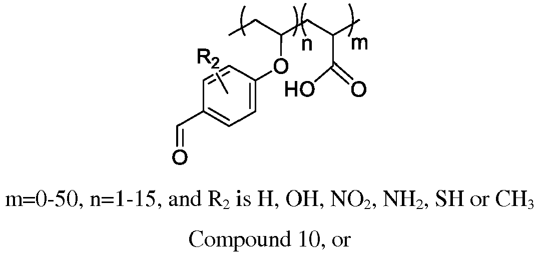 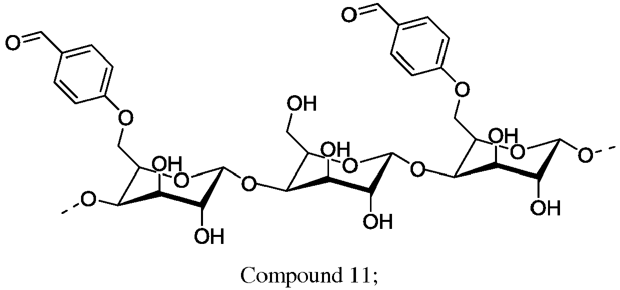 or
b) a solid surface.

2. The malodor counteractant compound of claim 1, wherein the solid surface comprises a silica or clay surface.

3. A consumer, industrial or textile product, which is a consumer or industrial product comprising a malodor counteractant compound wherein the malodor counteractant compound is a compound of claim 1 or 2, or which is a textile product comprising a malodor counteractant compound comprising a benzaldehyde covalently attached to a polymer, a surfactant or a solid surface, wherein the polymer comprises a polyol, polysaccharide, polyamine, polyacrylate, alkene oxide polymer with OH or NH₂ end groups, or block or random copolymer thereof; wherein the surfactant comprises a poloxamine or an unbranched C13-C15 oxo alcohol; or wherein the solid surface comprises a silica or clay surface.

4. The consumer, industrial or textile product of claim 3, wherein the malodor counteractant compound has the structure: 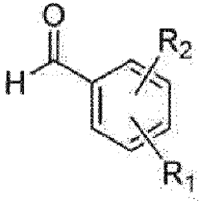 wherein
R₁ is the polymer, the surfactant, or the solid surface; and
R₂ is H, OH, NO₂, NH₂, SH or CH₃.

5. A method for counteracting an amine-based malodor of a consumer, industrial or textile product or the surrounding environment thereof comprising the step of adding a malodor counteractant compound to the product so that the amine-based malodor of a consumer, industrial or textile product or the surrounding environment thereof is counteracted, wherein the malodor counteractant compound comprises a benzaldehyde covalently attached to a polymer, a surfactant or a solid surface, wherein the polymer comprises a polyol, polysaccharide, polyamine, polyacrylate, alkene oxide polymer with OH or NH₂ end groups, or block or random copolymer thereof.

6. The method of claim 5, wherein the malodor counteractant compound is a compound of claim 1 or 2.

7. The method of claim 5, wherein the malodor counteractant compound has the structure: 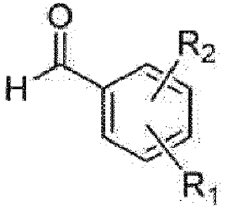 wherein
R₁ is a polymer, a surfactant, or a solid surface; and R₂ is H, OH, NO₂, NH₂, SH or CH₃,
wherein the polymer comprises a polyol, polysaccharide, polyamine, polyacrylate, alkene oxide polymer with OH or NH2 end groups, or block or random copolymer thereof.

## Patentansprüche

1. Schlechtem Geruch entgegenwirkende Verbindung, umfassend einen Benzaldehyd, der kovalent an:
a) ein Polymer gebunden ist, wobei es sich bei der Verbindung um 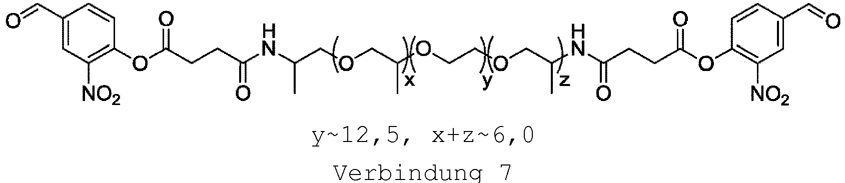 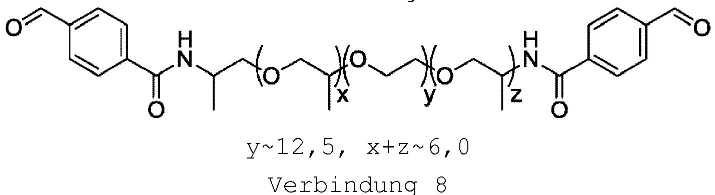 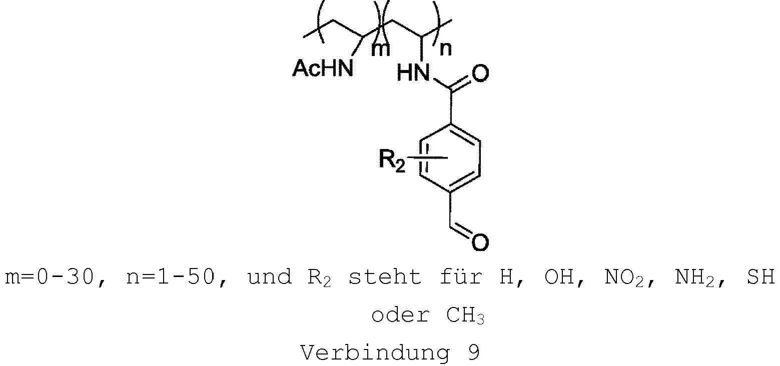 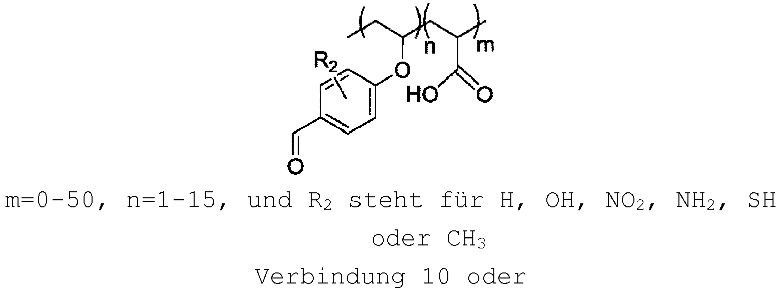 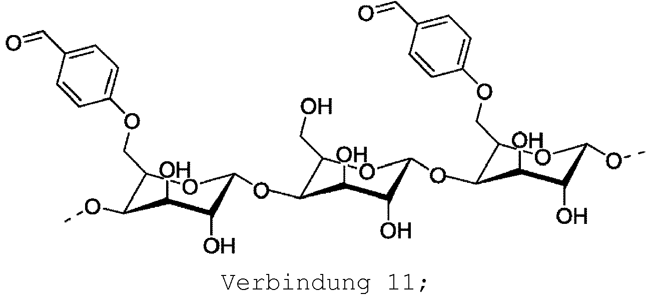 handelt, oder
b) eine feste Oberfläche gebunden ist.

2. Schlechtem Geruch entgegenwirkende Verbindung nach Anspruch 1, wobei die feste Oberfläche eine Siliciumdioxid- oder Tonoberfläche umfasst.

3. Konsumprodukt, technisches Produkt oder Textilprodukt, bei dem es sich um ein Konsumprodukt oder technisches Produkt handelt, das eine schlechtem Geruch entgegenwirkende Verbindung umfasst, wobei es sich bei der schlechtem Geruch entgegenwirkenden Verbindung um eine Verbindung nach Anspruch 1 oder 2 handelt, oder bei dem es sich um ein Textilprodukt handelt, das eine schlechtem Geruch entgegenwirkende Verbindung umfasst, die einen Benzaldehyd umfasst, der kovalent an ein Polymer, ein Tensid oder eine feste Oberfläche gebunden ist, wobei das Polymer ein Polyol, Polysaccharid, Polyamin, Polyacrylat, Alkenoxid-Polymer mit OH- oder NH₂-Endgruppen oder ein blockartig oder statistisch aufgebautes Copolymer davon umfasst; wobei das Tensid ein Poloxamin oder einen unverzweigten C13-C15-Oxo-Alkohol umfasst oder wobei die feste Oberfläche eine Siliciumdioxid- oder Tonoberfläche umfasst.

4. Konsumprodukt, technisches Produkt oder Textilprodukt nach Anspruch 3, wobei die schlechtem Geruch entgegenwirkende Verbindung die Struktur: 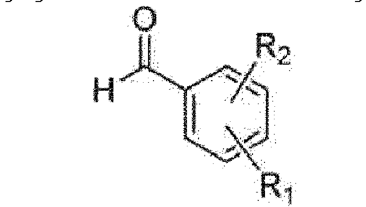 aufweist, wobei
R₁ für das Polymer, das Tensid oder die feste Oberfläche steht und
R₂ für H, OH, NO₂, NH₂, SH oder CH₃ steht.

5. Verfahren zum Entgegenwirken gegen einen auf Amin basierenden schlechten Geruch eines Konsumprodukts, technischen Produkts oder Textilprodukts oder der Umgebung davon, bei dem man eine schlechtem Geruch entgegenwirkende Verbindung zu dem Produkt gibt, so das dem auf Amin basierenden schlechten Geruch eines Konsumprodukts, technischen Produkts oder Textilprodukts oder der Umgebung davon entgegengewirkt wird, wobei die schlechtem Geruch entgegenwirkende Verbindung einen Benzaldehyd umfasst, der kovalent an ein Polymer, ein Tensid oder eine feste Oberfläche gebunden ist, wobei das Polymer ein Polyol, Polysaccharid, Polyamin, Polyacrylat, Alkenoxid-Polymer mit OH- oder NH₂-Endgruppen oder ein blockartig oder statistisch aufgebautes Copolymer davon umfasst.

6. Verfahren nach Anspruch 5, bei dem es sich bei der schlechtem Geruch entgegenwirkenden Verbindung um eine Verbindung nach Anspruch 1 oder 2 handelt.

7. Verfahren nach Anspruch 5, bei dem die schlechtem Geruch entgegenwirkende Verbindung die Struktur: 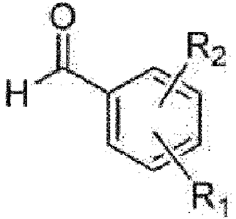 aufweist, wobei
R₁ für ein Polymer, ein Tensid oder eine feste Oberfläche steht und R₂ für H, OH, NO₂, NH₂, SH oder CH₃ steht,
wobei das Polymer ein Polyol, Polysaccharid, Polyamin, Polyacrylat, Alkenoxid-Polymer mit OH- oder NH₂-Endgruppen oder ein blockartig oder statistisch aufgebautes Copolymer davon umfasst.

## Revendications

1. Composé neutralisant les mauvaises odeurs comprenant un benzaldéhyde lié de façon covalente à :
a) un polymère, le composé étant 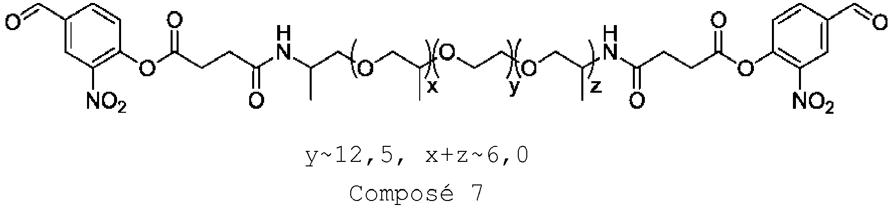 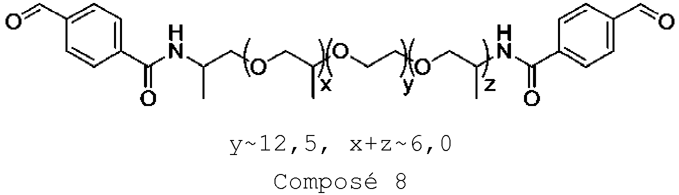 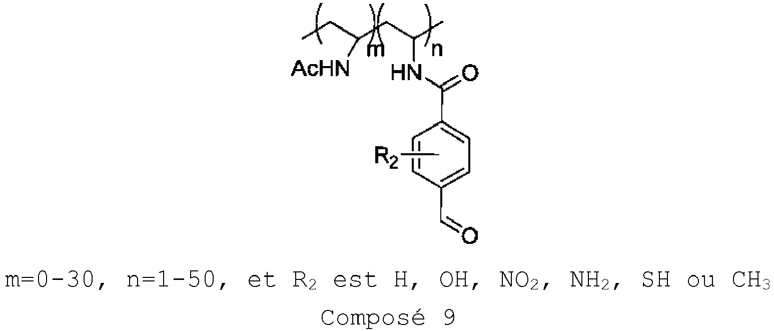 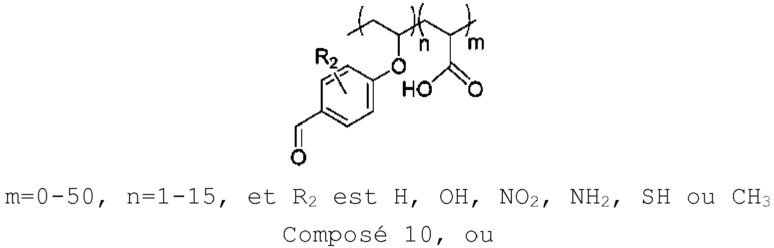 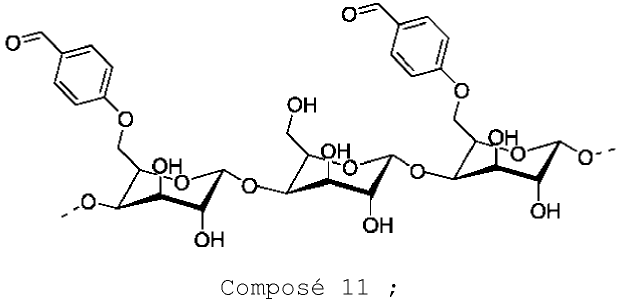 ou
b) une surface solide.

2. Composé neutralisant les mauvaises odeurs de la revendication 1, dans lequel la surface solide comprend une surface de silice ou d'argile.

3. Produit de consommation, industriel ou textile, qui est un produit de consommation ou industriel comprenant un composé neutralisant les mauvaises odeurs dans lequel le composé neutralisant les mauvaises odeurs est un composé de la revendication 1 ou 2, ou qui est un produit textile comprenant un composé neutralisant les mauvaises odeurs comprenant un benzaldéhyde lié de façon covalente à un polymère, un tensioactif ou une surface solide, dans lequel le polymère comprend un polyol, un polysaccharide, une polyamine, un polyacrylate, un polymère d'oxyde d'alcène avec des groupes terminaux OH ou NH₂, ou un copolymère séquencé ou statistique de ceux-ci ; dans lequel le tensioactif comprend une poloxamine ou un oxo-alcool en C13-C15 non ramifié ; ou dans lequel la surface solide comprend une surface de silice ou d'argile.

4. Produit de consommation, industriel ou textile de la revendication 3, dans lequel le composé neutralisant les mauvaises odeurs a la structure : 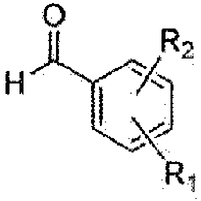 dans laquelle
R₁ est le polymère, le tensioactif, ou la surface solide ; et R₂ est H, OH, NO₂, NH₂, SH ou CH₃.

5. Procédé de neutralisation d'une mauvaise odeur à base d'amine d'un produit de consommation, industriel ou textile ou de l'environnement périphérique de celui-ci comprenant l'étape d'ajout d'un composé neutralisant les mauvaises odeurs au produit de sorte que la mauvaise odeur à base d'amine d'un produit de consommation, industriel ou textile ou de l'environnement périphérique de celui-ci soit neutralisée, dans lequel le composé neutralisant les mauvaises odeurs comprend un benzaldéhyde lié de façon covalente à un polymère, un tensioactif ou une surface solide, dans lequel le polymère comprend un polyol, un polysaccharide, une polyamine, un polyacrylate, un polymère d'oxyde d'alcène avec des groupes terminaux OH ou NH₂, ou un copolymère séquencé ou statistique de ceux-ci.

6. Procédé de la revendication 5, dans lequel le composé neutralisant les mauvaises odeurs est un composé de la revendication 1 ou 2.

7. Procédé de la revendication 5, dans lequel le composé neutralisant les mauvaises odeurs a la structure : 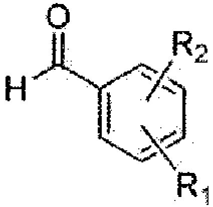 dans laquelle
R₁ est un polymère, un tensioactif, ou une surface solide ; et R₂ est H, OH, NO_{2,} NH₂, SH ou CH₃,
dans lequel le polymère comprend un polyol, un polysaccharide, une polyamine, un polyacrylate, un polymère d'oxyde d'alcène avec des groupes terminaux OH ou NH₂, ou un copolymère séquencé ou statistique de ceux-ci.
